# EUROPEAN PATENT APPLICATION

(11) **EP 4 509 081 A2**
(43) Date of publication of application: **19.02.2025**
(21) Application number: 24194243.2
(22) Date of filing: 13.08.2024
(51) Int. Cl.: A61B 34/10, A61F 2/40, A61B 34/00

(54) **ORTHOPAEDIC IMPLANT SYSTEMS AND ASSOCIATED METHODS OF FIXATION**

(30) Priority: 16.08.2023 US 202318450502
(71) Applicant: Arthrex, Inc, Naples, FL 34108-1945 (US)
(72) Inventor: METCALFE, Nick, Bonita Springs, FL, 34134 (US); DELEON, Steven Jim, San Antonio, TX, 78232 (US)
(74) Representative: Lohr, Jöstingmeier & Partner

(57) **Abstract**

This disclosure relates to surgical systems and methods for planning and implementing surgical procedures. The systems and methods disclosed herein may be utilized for implant design and placement. The disclosed implants may include one or more fixation members for securing the respective implant at a surgical site. The implant design may establish a geometry of each fixation member.

## Description

### BACKGROUND

This disclosure relates to surgical systems and methods for planning and implementing surgical procedures, and more particularly, to orthopaedic implant designs.

Many bones of the human musculoskeletal system include articular surfaces. The articular surfaces articulate relative to other bones to facilitate different types and degrees of joint movement. The articular surfaces can erode or experience bone loss over time due to repeated use or wear or can fracture as a result of a traumatic impact. These types of bone defects can cause joint instability and pain.

Bone deficiencies may occur along the articular surfaces of the glenoid bone. Some techniques utilize a bone graft and/or implant to fill a defect in the glenoid bone. The implant may include a central post insertable in a recess of the glenoid to secure the implant to the glenoid. The implant may also be secured to the glenoid utilizing one or more fasteners.

### SUMMARY

This disclosure relates to improved systems, devices and methods of planning and/or performing a surgical procedure. The systems may be utilized for designing an orthopaedic implant. The implant may incorporate one or more fixation members. The fixation member may be defined relative to one or more apertures extending through the implant and/or the geometry of an associated bone. The fixation member may be defined relative to a center of rotation established by the implant.

A system for planning a surgical procedure may include, *inter alia,* a computing device including a processor coupled to memory. The processor may be configured to access a virtual bone model from the memory. The virtual bone model may be associated with a bone of a patient. The processor may be configured to define a position of a virtual implant model relative to the virtual bone model. The implant model may include a main body extending along an implant axis between a front face and a rear face. The implant model may include one or more peripheral apertures extending through the main body. The one or more peripheral apertures may be dimensioned to receive a respective fastener. The implant model may include at least one fixation member that may extend from the rear face. The processor may be configured to define a geometry of the at least one fixation member, which may include defining a position of the at least one fixation member relative to the main body in response to moving the at least one fixation member along a reference plane transverse to the implant axis such that a volume of the at least one fixation member may be spaced apart from a combined volume of the one or more peripheral apertures. The processor may be configured to generate a configuration associated with a physical implant model that may be representative of the virtual implant model according to the defined geometry. Such a configuration may be held in a configuration file and it may be configured to be used by a manufacturing machine, e.g. an additive manufacturing machine. Such a configuration may be a configuration of/for a manufacturing machine.

A system for planning a surgical procedure may include, *inter alia,* a computing device including a processor coupled to memory. The processor may be configured to access a virtual bone model from the memory. The virtual bone model may be associated with a bone of a patient. The processor may be configured to define a position of a virtual implant model relative to the virtual bone model. The virtual implant model may include one or more peripheral apertures that may extend through a main body. The implant model may include a fixation member that may extend from a rear face of the main body. The processor may be configured to define a length of the fixation member such that a distal end portion of the fixation member may perforate first and second faces on opposite sides of a volume of the virtual bone model when the virtual implant model may be situated in the defined position. The processor may be configured to generate a configuration associated with a physical implant model that may be representative of the virtual implant model according to the defined length.

A method of establishing an implant for a surgical procedure may include, *inter alia,* accessing a virtual bone model from memory. The virtual bone model may be associated with a bone of a patient. The method may include defining a position of a virtual implant model relative to the virtual bone model. The virtual implant model may include a main body extending between a front face and a rear face. The implant model may include one or more peripheral apertures that may extend through the main body. The one or more peripheral apertures may be dimensioned to receive a respective fastener. The implant model may include at least one fixation member that may extend from the rear face. The method may include defining a geometry of the at least one fixation member, which may include defining a position of the at least one fixation member relative to the main body in response to moving the at least one fixation member along a reference plane such that a volume of the at least one fixation member may be spaced apart from a combined volume of the one or more peripheral apertures. The method may include generating a configuration associated with a physical implant that may be representative of the virtual implant model according to the defined geometry.

An orthopaedic implant for a surgical procedure may be configured to mate with an articulation component to establish a center of rotation that may be associated with an adjacent implant or bone. The implant may include, *inter alia,* a main body that may extend along an implant axis between a front face and a rear face. The main body may include one or more peripheral apertures that may extend through the main body and may be dimensioned to receive a respective fastener to secure the main body to bone. The rear face may be dimensioned to substantially follow a contour of an articular surface of a bone. The implant may include at least one fixation member extending from the rear face along a fixation axis. The at least one fixation member may be spaced apart from the one or more peripheral apertures. The at least one fixation member may be dimensioned such that the fixation axis may be offset from the center of rotation.

An orthopaedic implant for a surgical procedure may include, *inter alia,* a main body that may extend along an implant axis between a front face and a rear face. The main body may include one or more peripheral apertures that may extend through the main body and may be dimensioned to receive a respective fastener to secure the main body to bone. The rear face may be dimensioned to substantially follow a contour of an articular surface of a bone. The main body may be dimensioned to mate with an articulation component to establish a center of rotation that may be associated with an adjacent implant or bone. At least one fixation member may be securable in bone. The at least one fixation member may extend along a fixation axis. The at least one fixation member may be spaced apart from the one or more peripheral apertures. The at least one fixation member may be dimensioned such that the fixation axis may be offset from the center of rotation.

The present disclosure may include any one or more of the individual features disclosed above and/or below alone or in any combination thereof.

The various features and advantages of this disclosure will become apparent to those skilled in the art from the following detailed description. The drawings that accompany the detailed description can be briefly described as follows.

### BRIEF DESCRIPTION OF THE DRAWINGS

Figure 1 discloses a planning system according to an implementation.
Figure 2 discloses a planning system including a user interface.
Figures 3A-3F disclose an orthopaedic implant.
Figures 4-5 disclose implementations of an implant.
Figures 6A-6D disclose an implant model positioned relative to a bone model.
Figures 7 and 8A-8B disclose a fixation member at various positions relative to an implant model in a display window of the user interface of Figure 2.
Figures 9-10 disclose an implant model including a fixation member arranged at various positions relative to a bone model in display windows of the user interface of Figure 2.
Figures 11 and 12A-12F disclose an implant model including a fixation member arranged relative to surfaces of a bone model in display windows of the user interface of Figure 2.
Figures 13-14 disclose implant models including fixation members relative to a center of rotation of the respective implant model in display windows of the user interface of Figure 2.
Figure 15 discloses a method of designing an orthopaedic implant according to an implementation.

Like reference numbers and designations in the various drawings indicate like elements.

### DETAILED DESCRIPTION

This disclosure relates to implants and planning methods for performing a surgical procedure. The implants described herein may be utilized during arthroplasty procedures for restoring functionality to joints having advanced cartilage disease and other defects. The disclosed systems and methods may be utilized to establish an implant design. The implant design may be utilized to form a physical implant for treating the patient. The implant may incorporate one or more fixation members for securing the implant to bone or other tissue. The surgeon or clinical user may interact with the system to select or otherwise define a position and/or dimensions(s) of each fixation member to achieve improved fixation based on a geometry of the bone, bone quality, bone density and other characteristics. Utilizing the techniques disclosed herein, the fixation member may achieve bi-cortical penetration, which may improve fixation and reduce a likelihood of loosening of the implant after implantation in the patient. The position of the fixation member and a center of rotation established by the implant may be defined independently, which may provide flexibility to the surgeon in treating the patient and improve range of motion.

A system for planning a surgical procedure may include, *inter alia,* a computing device including a processor coupled to memory. The processor may be configured to access a virtual bone model from the memory. The virtual bone model may be associated with a bone of a patient. The processor may be configured to define a position of a virtual implant model relative to the virtual bone model. The implant model may include a main body extending along an implant axis between a front face and a rear face. The implant model may include one or more peripheral apertures extending through the main body. The one or more peripheral apertures may be dimensioned to receive a respective fastener. The implant model may include at least one fixation member that may extend from the rear face. The processor may be configured to define a geometry of the at least one fixation member, which may include defining a position of the at least one fixation member relative to the main body in response to moving the at least one fixation member along a reference plane transverse to the implant axis such that a volume of the at least one fixation member may be spaced apart from a combined volume of the one or more peripheral apertures. The processor may be configured to generate a configuration associated with a physical implant model that may be representative of the virtual implant model according to the defined geometry.

In any implementations, the processor may be configured to define a geometry of the virtual implant model such that the rear face may be dimensioned to substantially follow a contour of an articular surface associated with the virtual bone model.

In any implementations, the processor may be configured to define a trajectory associated with the fastener such that a volume of the fastener may be offset from the volume of the at least one fixation member. The processor may be configured to generate a surgical plan associated with the defined position of the virtual implant model and the defined trajectory.

In any implementations, the processor may be configured to cause the virtual implant model to be displayed in a graphical user interface relative to the virtual bone model. The processor may be configured to set a position of the main body relative to the virtual bone model. The processor may be configured to move the at least one fixation member relative to the reference plane in response to user interaction with the graphical user interface.

In any implementations, the processor may be configured to adjust a length of the at least one fixation member relative to the rear face of the main body.

In any implementations, the bone may be a scapula. The processor may be configured to adjust the length such that a free end of the at least one fixation member may perforate an anterior face and a posterior face that may cooperate to bound a volume that may be associated with a glenoid vault of the scapula.

In any implementations, the processor may be configured to generate at least one indicator associated with a first portion of the free end that may perforate the anterior face and/or a second portion of the free end that may perforate the posterior face.

In any implementations, the at least one indicator may include a first indicator associated with a first percentage of a periphery of the at least one fixation member that may perforate the anterior face and/or a second indicator associated with a second percentage of the periphery that may perforate the posterior face.

In any implementations, the at least one fixation member may extend along a fixation axis. The processor may be configured to position a virtual articulation component relative to the virtual implant model to establish a center of rotation that may be associated with an adjacent implant or bone that may mate with an articular surface of the virtual articulation component. The processor may be configured to define the position of the at least one fixation member such that the fixation axis may be offset from the center of rotation.

In any implementations, the at least one fixation member may include a first fixation member and a second fixation member. The processor may be configured to move the first and second fixation members independently of each other relative to the reference plane such that the first and second fixation members may be spaced apart.

In any implementations, the processor may be configured to adjust a length of the first fixation member and a length of the second fixation member independently of each other such that a free end of the first fixation member and/or a free end of the second fixation member may perforate a cortical wall associated with the virtual bone model.

In any implementations, the articular surface may have a substantially convex geometry. A perimeter of the main body may have a substantially circular geometry.

A system for planning a surgical procedure may include, *inter alia,* a computing device including a processor coupled to memory. The processor may be configured to access a virtual bone model from the memory. The virtual bone model may be associated with a bone of a patient. The processor may be configured to define a position of a virtual implant model relative to the virtual bone model. The virtual implant model may include one or more peripheral apertures that may extend through a main body. The implant model may include a fixation member that may extend from a rear face of the main body. The processor may be configured to define a length of the fixation member such that a distal end portion of the fixation member may perforate first and second faces on opposite sides of a volume of the virtual bone model when the virtual implant model may be situated in the defined position. The processor may be configured to generate a configuration associated with a physical implant model that may be representative of the virtual implant model according to the defined length.

In any implementations, the processor may be configured to define a geometry of the virtual implant model such that the rear face may be dimensioned to substantially follow a contour of an articular surface associated with the virtual bone model.

In any implementations, the bone may be a scapula. The first and second faces may be anterior and posterior faces that may be associated with a glenoid vault of the scapula.

A method of establishing an implant for a surgical procedure may include, *inter alia,* accessing a virtual bone model from memory. The method may be executed on a computing device including a processor coupled to memory. The virtual bone model may be associated with a bone of a patient. The method may include defining a position of a virtual implant model relative to the virtual bone model. The virtual implant model may include a main body extending between a front face and a rear face. The implant model may include one or more peripheral apertures that may extend through the main body. The one or more peripheral apertures may be dimensioned to receive a respective fastener. The implant model may include at least one fixation member that may extend from the rear face. The method may include defining a geometry of the at least one fixation member, which may include defining a position of the at least one fixation member relative to the main body in response to moving the at least one fixation member along a reference plane such that a volume of the at least one fixation member may be spaced apart from a combined volume of the one or more peripheral apertures. The method may include generating a configuration associated with a physical implant that may be representative of the virtual implant model according to the defined geometry.

In any implementations, the method may include forming the physical implant based on the generated configuration.

In any implementations, the method may include blocking movement of the at least one fixation member within the combined volume of the one or more peripheral apertures.

In any implementations, the method may include defining a position of an articulation component relative to the virtual implant model to establish a center of rotation that may be associated with an adjacent implant or bone that may mate with an articular surface of the articulation component. The step of defining the position of the at least one fixation member may occur such that an axis of the at least one fixation member may be offset from the center of rotation.

In any implementations, the step of defining the geometry of the at least one fixation member may include defining a length of the at least one fixation member relative to the rear face of the main body such that a distal end portion of the at least one fixation member may perforate opposed first and second faces that may cooperate to bound the volume of the virtual bone model when the virtual implant model may be situated in the defined position.

In any implementations, the bone may be a scapula. The first and second faces may be anterior and posterior faces that may be associated with a glenoid vault of the scapula.

An orthopaedic implant for a surgical procedure may be configured to mate with an articulation component to establish a center of rotation that may be associated with an adjacent implant or bone. The implant may include, *inter alia,* a main body that may extend along an implant axis between a front face and a rear face. The main body may include one or more peripheral apertures that may extend through the main body and may be dimensioned to receive a respective fastener to secure the main body to bone. The rear face may be dimensioned to substantially follow a contour of an articular surface of a bone. The implant may include at least one fixation member extending from the rear face along a fixation axis. The at least one fixation member may be spaced apart from the one or more peripheral apertures. The at least one fixation member may be dimensioned such that the fixation axis may be offset from the center of rotation.

In any implementations, a first distance may be established between the fixation axis of the at least one fixation member and the center of rotation. A maximum width may be established across the main body. An offset ratio may be defined as the first distance divided by the maximum width. The main body and the at least one fixation member may be dimensioned such that the offset ratio may be equal to or greater than 0.10.

An orthopaedic implant for a surgical procedure may include, *inter alia,* a main body that may extend along an implant axis between a front face and a rear face. The main body may include one or more peripheral apertures that may extend through the main body and may be dimensioned to receive a respective fastener to secure the main body to bone. The rear face may be dimensioned to substantially follow a contour of an articular surface of a bone. The main body may be dimensioned to mate with an articulation component to establish a center of rotation that may be associated with an adjacent implant or bone. At least one fixation member may be securable in bone. The at least one fixation member may extend along a fixation axis. The at least one fixation member may be spaced apart from the one or more peripheral apertures. The at least one fixation member may be dimensioned such that the fixation axis may be offset from the center of rotation.

In any implementations, a first distance may be established between the fixation axis of the at least one fixation member and the center of rotation. A maximum width may be established across the main body. An offset ratio may be defined as the first distance divided by the maximum width. The offset ratio may be equal to or greater than 0.10

In any implementations, the at least one fixation member may be dimensioned to perforate a glenoid vault associated with a scapula.

An implant may further be described by the following clauses:
1. An orthopaedic implant for a surgical procedure, the implant configured to mate with an articulation component to establish a center of rotation associated with an adjacent implant or bone, the implant comprising:
   a main body extending along an implant axis between a front face and a rear face, and one or more peripheral apertures extending through the main body and dimensioned to receive a respective fastener to secure the main body to bone, wherein the rear face is dimensioned to substantially follow a contour of an articular surface of a bone; and
   at least one fixation member extending from the rear face along a fixation axis, wherein the at least one fixation member is spaced apart from the one or more peripheral apertures, and the at least one fixation member is dimensioned such that the fixation axis is offset from the center of rotation.
2. The implant as recited in clause 1, wherein:
   a first distance is established between the fixation axis of the at least one fixation member and the center of rotation, a maximum width is established across the main body, an offset ratio is defined as the first distance divided by the maximum width, and the main body and the at least one fixation member are dimensioned such that the offset ratio is equal to or greater than 0.10.
3. An orthopaedic implant for a surgical procedure, comprising:
   a main body extending along an implant axis between a front face and a rear face, and one or more peripheral apertures extending through the main body and dimensioned to receive a respective fastener to secure the main body to bone, wherein the rear face is dimensioned to substantially follow a contour of an articular surface of a bone, and the main body is dimensioned to mate with an articulation component to establish a center of rotation associated with an adjacent implant or bone; and
   at least one fixation member securable in bone, wherein the at least one fixation member extends along a fixation axis, the at least one fixation member is spaced apart from the one or more peripheral apertures, and the at least one fixation member is dimensioned such that the fixation axis is offset from the center of rotation.
4. The implant as recited in clause 3, wherein:
   a first distance is established between the fixation axis of the at least one fixation member and the center of rotation, a maximum width is established across the main body, an offset ratio is defined as the first distance divided by the maximum width, and the offset ratio is equal to or greater than 0.10.
5. The implant as recited in clause 3, wherein:
   the at least one fixation member is dimensioned to perforate a glenoid vault associated with a scapula.

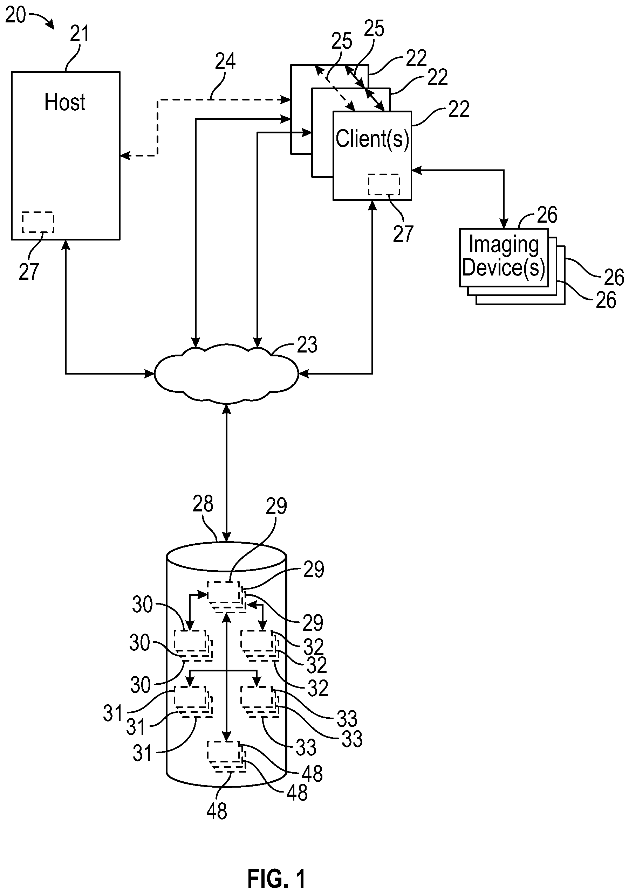

Figure 1 discloses a planning system 20 that may be utilized for planning surgical procedures. The system 20 may be used for planning orthopaedic procedures, including pre-operatively, intra-operatively and/or post-operatively to create, edit, execute and/or review surgical plans. The system 20 may be utilized for various orthopaedic and other surgical procedures, such as an arthroplasty to repair a joint. The system 20 may be utilized in the design and/or placement of an implant, such as an implant incorporated into a shoulder prosthesis. Although the planning systems and methods disclosed herein primarily refer to repair of a glenoid or humerus during an anatomic or reverse shoulder reconstruction, it should be understood that the planning system 20 may be utilized in the repair of other locations of the patient and other surgical procedures including repair of other bones and joints such as a wrist, hand, hip, knee or ankle.

The system 20 may include a host computer 21 and one or more client computers 22. The host computer 21 may be configured to execute one or more software programs. In implementations, the host computer 21 may be more than one computer jointly configured to process software instructions serially or in parallel.

The host computer 21 may be in communication with one or more networks such as a network 23 comprised of one or more computing devices. The network 23 may be a private local area network (LAN), a private wide area network (WAN), the Internet, or a mesh network, for example.

The host computer 21 and each client computer 22 may include one or more of a computer processor, memory, storage means, network device and input and/or output devices and/or interfaces. The input devices may include a keyboard, mouse, etc. The output device may include a monitor, speakers, printers, etc. The memory may, for example, include UVPROM, EEPROM, FLASH, RAM, ROM, DVD, CD, a hard drive, or other computer readable medium which may store data and/or other information relating to the planning and implementation techniques disclosed herein. The host computer 21 and each client computer 22 may be a desktop computer, laptop computer, smart phone, tablet, or any other computing device. The interface may facilitate communication with the other systems and/or components of the network 23.

Each client computer 22 may be configured to communicate with the host computer 21 directly via a direct client interface 24 or over the network 23. In another implementation, the client computers 22 may be configured to communicate with each other directly via a peer-to-peer interface 25.

The system 20 may include, or may be coupled to, one or more imaging devices 26. Each client computer 22 may be coupled to one or more imaging devices 26. Each imaging device 26 may be configured to capture or acquire one or more images 30 of patient anatomy residing within a scan field (e.g., window) of the imaging device 26. The imaging device 26 may be configured to capture or acquire two-dimensional (2D) and/or three-dimensional (3D) greyscale and/or color images 30. Various imaging devices 26 may be utilized, such as an X-ray machine, computerized tomography (CT) machine or magnetic resonance imaging (MRI) machine that may obtain one or more images of a patient.

The client computers 22 may be configured to execute one or more software programs, such as a various surgical tools. Each client computer 22 may be operable to access and locally and/or remotely execute a planning environment 27. The planning environment 27 may be a standalone software package or may be incorporated into another surgical tool. The planning environment 27 may be configured to communicate with the host computer 21 either over the network 23 or directly through the direct client interface 24.

The planning environment 27 may be configured to interact with one or more of the imaging devices 26 to capture or acquire images 30 of patient anatomy. The planning environment 27 may provide a display or visualization of one or more images 30, virtual bone models 31, virtual implant models 32 and/or virtual transfer models 48 via one or more graphical user interfaces (GUI). Each image 30, bone model 31, implant model 32, transfer model 48 and other data and information may be stored in one or more files or records according to a specified data structure.

The system 20 may include at least one storage system 28, which may be operable to store or otherwise provide data to other computing devices. The storage system 28 may be a storage area network device (SAN) configured to communicate with the host computer 21 and/or the client computers 22 over the network 23. In implementations, the storage system 28 may be incorporated within or directly coupled to the host computer 21 and/or client computers 22. The storage system 28 may be configured to store one or more of computer software instructions, data, database files, configuration information, etc.

In implementations, the system 20 may be a client-server architecture configured to execute computer software on the host computer 21, which may be accessible by the client computers 22 using either a thin client application or a web browser executing on the client computers 22. The host computer 21 may load the computer software instructions from local storage, or from the storage system 28, into memory and may execute the computer software using the one or more computer processors.

The system 20 may include one or more databases 29. The databases 29 may be stored at a central location, such as the storage system 28. In another implementation, one or more databases 29 may be stored at the host computer 21 and/or may be a distributed database provided by one or more of the client computers 22. Each database 29 may be a relational database configured to associate one or more images 30, bone models 31, implant models 32 and/or transfer models 48 to each other and/or a surgical plan 33. Each surgical plan 33 may be associated with the anatomy of a respective patient. Each image 30, bone model 31, implant model 32, transfer model 48 and surgical plan 33 may be assigned a unique identifier or database entry. The database 29 may be configured to store data and other information corresponding to the images 30, bone models 31, implant models 32, transfer models 48 and surgical plans 33 in one or more database records or entries, and/or may be configured to link or otherwise associate one or more files corresponding to each respective image 30, bone model 31, implant model 32, transfer model 48 and surgical plan 33. Images 30, bone models 31, implant models 32, transfer models 48 and associated surgical plans 33 stored in the database(s) 29 may correspond to respective patient anatomies from prior surgical cases, and may be arranged into one or more predefined categories such as sex, age, ethnicity, defect category, procedure type, surgeon, facility or organization, etc.

Each image 30 and bone model 31 may include data and other information obtained from one or more medical devices or tools, such as the imaging devices 26. The bone model 31 may include coordinate information relating to an anatomy of the patient obtained or derived from image(s) 30 captured or otherwise obtained by the imaging device(s) 26. Each implant model 32 and transfer model 48 may include geometry and/or coordinate information associated with a predefined design or a design established or modified by the planning environment 27. The planning environment 27 may incorporate and/or interface with one or more modeling packages, such as a computer aided design (CAD) package, to render the models 31, 32, 48 as two-dimensional (2D) and/or three-dimensional (3D) volumes or constructs, which may overlay one or more of the images 30 in a display screen of a GUI.

The implant models 32 may correspond to implants and components of various shapes and sizes. Each implant may include one or more components that may be situated at a surgical site including screws, anchors and/or grafts. Each implant model 32 may correspond to a single component or may include two or more components that may be configured to establish an assembly. Each implant and associated component(s) may be formed of various materials, including metallic and/or non-metallic materials. Each bone model 31, implant model 32 and transfer model 48 may correspond to 2D and/or 3D geometry and may be utilized to generate a wireframe, mesh and/or solid construct in a display.

Each surgical plan 33 may be associated with one or more of the images 30, bone models 31, implant models 32 and/or transfer models 48. The surgical plan 33 may include various parameters associated with the images 30, bone models 31, implant models 32 and/or transfer models 48. The surgical plan 33 may include parameters relating to bone density and bone quality associated with patient anatomy captured in the image(s) 30. The surgical plan 33 may include parameters including spatial information relating to relative positioning and coordinate information of the selected bone model(s) 30, implant model(s) 32 and/or transfer model(s) 48.

The surgical plan 33 may include one or more revisions to a bone model 31 and information relating to a position of an implant model 32 and/or transfer model 48 relative to the original and/or revised bone model 31. The surgical plan 33 may include coordinate information relating to the revised bone model 31 and a relative position of the implant model 32 and/or transfer model 48 in predefined data structure(s). The planning environment 27 may be configured to make one or more revisions to a transfer model 48 automatically or in response to user interaction with the user interface. Revisions to each bone model 31, implant model 32, transfer model 48 and/or surgical plan 33 may be stored in the database 29 automatically and/or in response to user interaction with the system 20.

One or more surgeons and other users may be provided with a planning environment 27 via the client computers 22 and may simultaneously access each image 30, bone model 31, implant model 32, transfer model 48 and surgical plan 33 stored in the database(s) 29. Each user may interact with the planning environment 27 to create, view and/or modify various aspects of the surgical plan 33. Each client computer 22 may be configured to store local instances of the images 30, bone models 31, implant models 32, transfer models 48 and/or surgical plans 33, which may be synchronized in real-time or periodically with the database(s) 29. The planning environment 27 may be a standalone software package executed on a client computer 22 or may be provided as one or more services executed on the host computer 21.

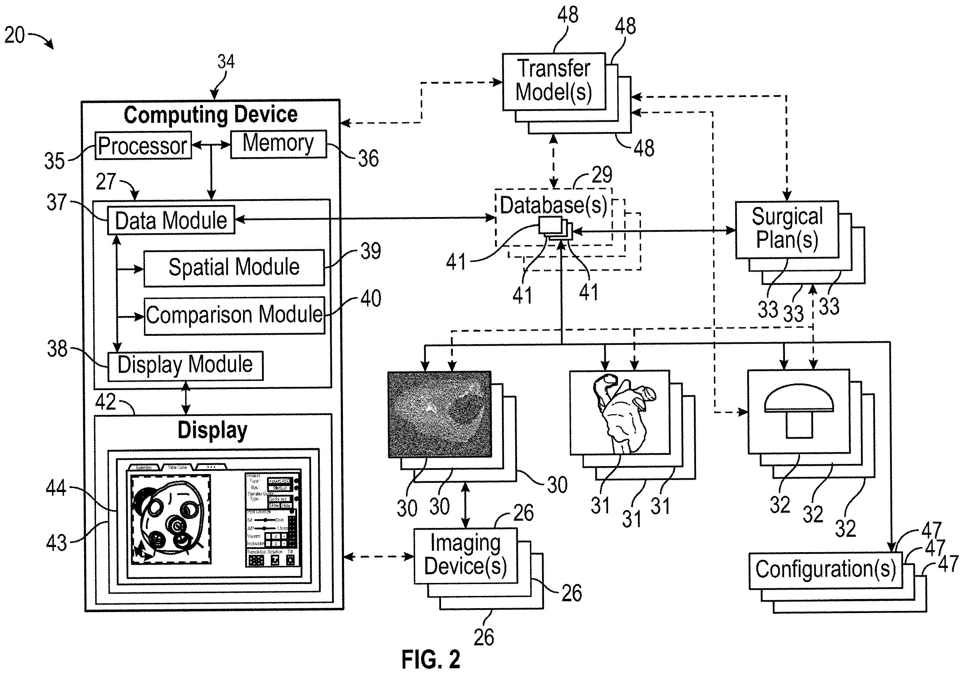

Referring to Figure 2, with continuing reference to Figure 1, the system 20 may include a computing device 34 including at least one processor 35 coupled to memory 36. The computing device 34 may include any of the computing devices disclosed herein, including the host computer 21 and/or client computer 22. The processor 35 may be configured to execute a planning environment 27 for creating, editing, executing and/or reviewing one or more surgical plans 33 and any associated bone models 31, implant models 32 and transfer models 48 during pre-operative, intra-operative and/or post-operative phases of a surgery.

The planning environment 27 may include a data module 37, a display module 38, a spatial module 39 and a comparison module 40. Although four modules are shown, it should be understood that fewer or more than four modules may be utilized and/or one or more of the modules may be combined to provide the disclosed functionality.

The data module 37 may be configured to access, retrieve and/or store data and other information in the database(s) 29 corresponding to one or more images 30 of patient anatomy, bone model(s) 31, implant model(s) 32, transfer model(s) 48 and/or surgical plan(s) 33. The data and other information may be stored in one or more databases 29 as one or more records or entries 41. In implementations, the data and other information may be stored in one or more files that may be accessible by referencing one or more objects or memory locations referenced by the records 41.

The memory 36 may be configured to access, load, edit and/or store instances of one or more images 30, bone models 31, implant models 32, transfer models 48 and/or surgical plans 33 in response to one or more commands from the data module 37. The data module 37 may be configured to access a virtual bone model 31 from memory, such as the memory 36 and/or storage system 28. The bone model 31 may be associated with a bone of a patient. The data module 37 may be configured to cause the memory 36 to store a local instance of the image(s) 30, bone model(s) 31, implant model(s) 32, transfer model(s) 48 and/or surgical plan(s) 33, which may be synchronized with the records 41 in the database(s) 29.

The data module 37 may be configured to receive data and other information corresponding to at least one or more images 30 of patient anatomy from various sources such as the imaging device(s) 26. The data module 37 may be configured to command the imaging device 26 to capture or acquire the images 30 automatically or in response to user interaction.

The display module 38 may be configured to display data and other information relating to one or more surgical plans 33 in at least one graphical user interface (GUI) 43, including one or more of the images 30, bone models 31, implant models 32 and/or transfer models 48. The computing device 34 may incorporate, or may be coupled to, a display device 42. The user interface 43 may include one or more display windows 44. The display module 38 may be configured to cause the display device 42 to display information in the display window(s) 44 and/or another portion of the user interface 43. A surgeon or other user may interact with the user interface 43 via the planning environment 27 to view one or more images 30 of patient anatomy and/or any associated bone models 31, implant models 32 and transfer models 48. The surgeon or other user may interact with the user interface 43 via the planning environment 27 to create, edit, execute and/or review one or more surgical plans 33.

The planning system 20 may be configured to access, generate, review, edit and/or approve one or more configurations 47 associated with respective physical implant(s). The physical implant may include any of the implants disclosed herein. The physical implant may be representative of a respective virtual implant model 32. The configuration 47 may specify various information for forming an instance of a physical implant, which may be based on a respective virtual implant model 32. The configuration 47 may include one or more files in a predetermined data structure or format. In implementations, the configuration 47 may include a coordinate set and/or other information such as material selection(s) associated with volume(s) of the physical implant. Each physical implant may be formed utilizing various techniques, including any of the techniques disclosed herein such as rapid prototyping (e.g., printing) and other additive manufacturing techniques, casting, machining, etc.

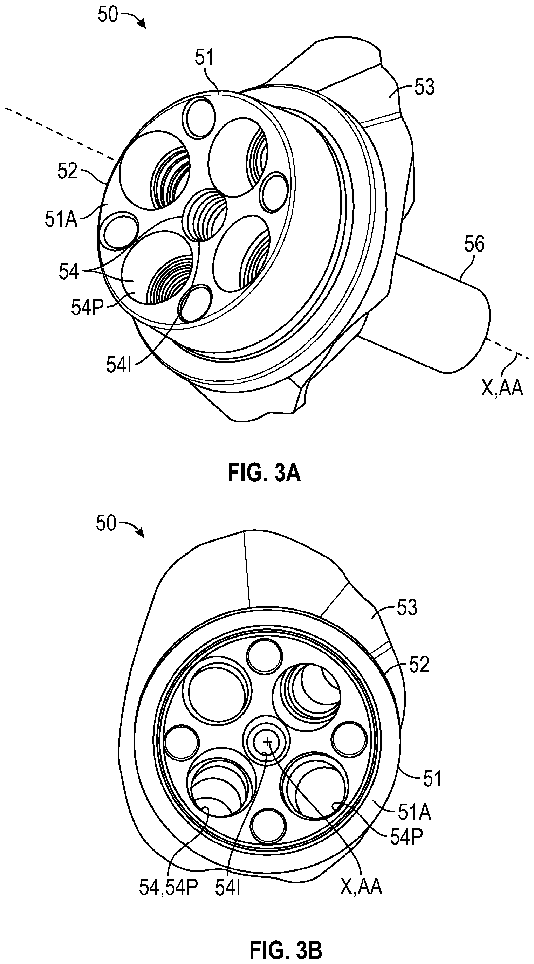

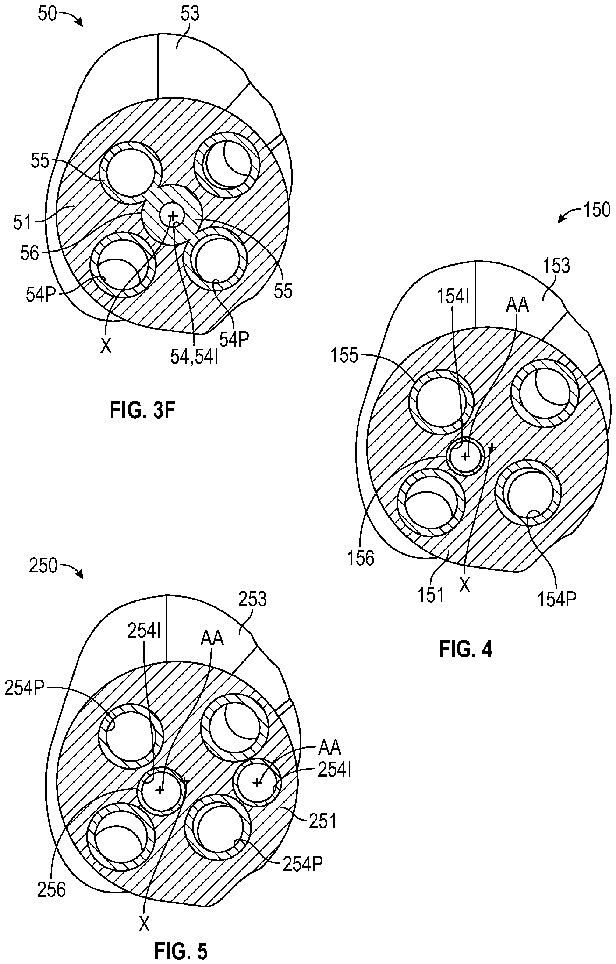

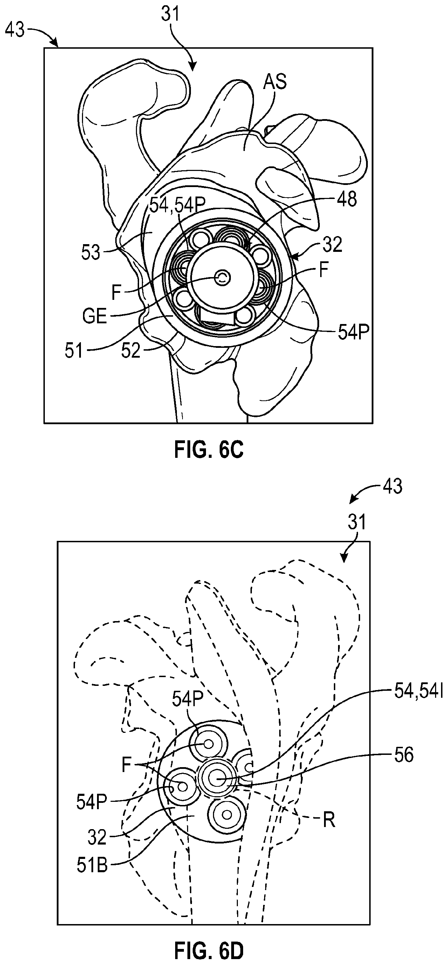

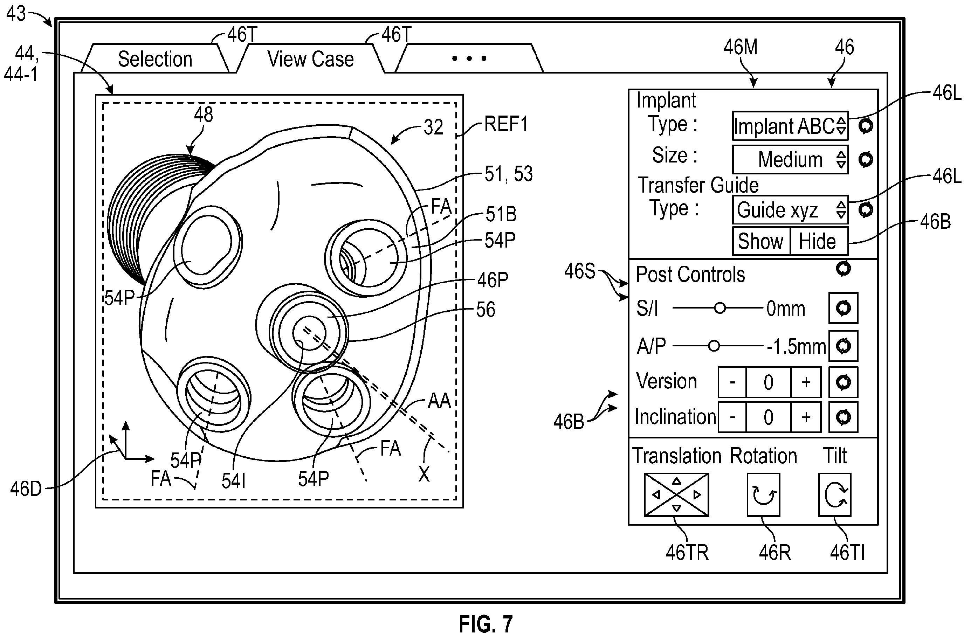

Referring to Figures 3A-3F, with continuing reference to Figure 2, an orthopaedic implant 50 according to an implementation is disclosed. The implant 50 may be a physical implant or may be an implant model associated with a physical implant. The implant 50 may be configured to abut or contact bone or other tissue. The bone may be a portion of a glenoid or another bone associated with a joint of a patient, including any of the bones and joints disclosed herein. In implementations, the implant 50 may be utilized in an anatomical and/or reverse shoulder reconstruction. The bone may be associated with a respective bone model 31. The implant 50 may be configured to interface with a transfer guide. A surgeon or clinical user may position and orient the implant 50 based on a position of the transfer guide relative to tissue such as bone. The transfer guide may be associated with a respective transfer model 48 (see, e.g., Figures 6C and 7). The transfer model 48 may be dimensioned to at least partially receive or otherwise interface with a guide element GE, which may be insertable in bone or other tissue at the surgical site (shown in dashed lines in Figure 6C). The transfer model 48 may interface with the guide element GE to establish a position and/or orientation of an associated implant relative to the bone or other tissue according to a surgical plan 33. The planning environment 27 may be configured to establish a geometry of the transfer model 48 based on a position and orientation of the guide element GE and a geometry of the associated implant model 32 for implementing the surgical plan 33.

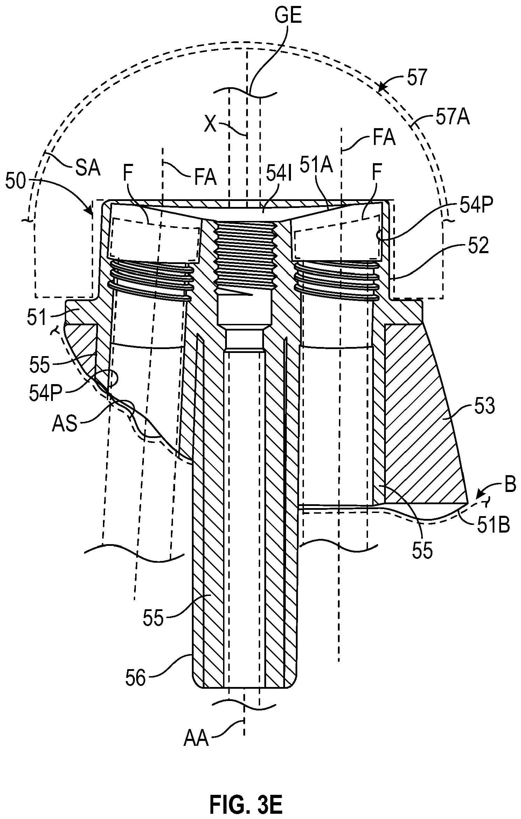

The implant 50 may include a main body 51 dimensioned to abut bone at a surgical site (see, e.g., bone B of Figure 3E). The main body 51 may extend along an implant (e.g., longitudinal) axis X between a first (e.g., front) face 51A and a second (e.g., rear) face 51B generally opposed to the first face 51A. The rear face 51B may be dimensioned to contact bone or other tissue to seat the implant 50 at the surgical site.

In the implementation of Figures 3E and 6C-6D, the main body 51 may be dimensioned to receive one or more fasteners F (shown in dashed lines in Figure 3E). Each of the fasteners F may be dimensioned to be at least partially received in bone to secure the implant 50 at the surgical site. Various fasteners may be utilized with the implant 50, such as compression screws, nails, pins, etc. suitable for securing the implant 50 to bone or other tissue at the surgical site.

The implant 50 may include a baseplate 52 and augment 53 that may establish the main body 51. The augment 53 may extend outwardly from the baseplate 52 relative to the axis X. The baseplate 52 may establish the front face 51A of the main body 51. The augment 53 may establish the rear face 51B of the main body 51. The baseplate 52 and augment 53 may be integrally formed to establish a monolithic or unitary component or may be separate and distinct components that may be fixedly attached or otherwise secured to each other. The baseplate 52 and augment 53 may be formed utilizing any of the techniques disclosed herein, such as a casting or additive manufacturing technique. In implementations, the augment 53 may be omitted.

The baseplate 52 and augment 53 may have various geometries. A perimeter of the baseplate 52 and/or another portion of the main body 51 may have a substantially circular or elliptical geometry, although other geometries may be utilized. For the purposes of this disclosure, the terms "substantially," "approximately" and "about" mean ±10 percent of the stated value or relationship unless otherwise indicated. The augment 53 may be dimensioned to contact bone. The rear face 51B and/or other portions of the main body 51 may be dimensioned to approximate a geometry of a bone defect or may have one or more surfaces having a patient-specific geometry dimensioned to substantially conform to or otherwise follow a surface contour of the bone associated with a respective patient. The surface contour may be established along an articular surface of the respective bone. The rear face 51B of the implant 50 may be dimensioned to substantially follow a surface contour of a bone (see, e.g., bone B of Figure 3E). In implementations, the planning environment 27 may be configured to define a geometry of a virtual implant model 32 associated with the implant 50 such that the rear face 51B may have a patient-specific contour dimensioned to substantially follow a contour of an articular surface AS associated with the virtual bone model 31 (shown in dashed lines in Figure 3E). In other implementations, the rear face 51B of the implant 50 may include one or more regions having a substantially planar or other non-patient specific geometry.

The implant 50 may include one or more apertures 54. The apertures 54 may extend through the main body 51 of the implant 50, including the baseplate 52 and/or augment 53. The apertures 54 may be passages that extend between the front face 51A and rear face 51B of the implant 50. In the implementation of Figure 3E, each aperture 54 may be dimensioned to receive a respective fastener F to secure the implant 50 to bone or other tissue at the surgical site. Each fastener F may extend along a respective fastener axis FA. The planning environment 27 may be configured to establish a trajectory of each fastener axis FA and respective aperture 54 of the associated implant model 32.

One or more of the apertures 54 may be dimensioned to receive an instrument or other surgical device. The instrument may be a guide element GE such as a Kirschner wire or Steinman pin for positioning the implant 50 at the surgical site (see, e.g., guide element GE of Figures 3E and 6C). The planning environment 27 may be configured to establish a trajectory of the guide element GE. A physical transfer guide associated with the transfer model 48 may be dimensioned to establish a position and/or orientation of an implant associated with the guide element GE. In implementations, the apertures 54 may include an intermediate (e.g., central) aperture 541 and/or one or more peripheral apertures 54P. The peripheral apertures 54P may be circumferentially distributed about the implant axis X (see, e.g., Figure 3B).

The implant 50 may include one or more internal walls 55 that establish the respective apertures 54. In implementations, the internal walls 55 may have a generally tubular geometry. The augment 53, including portions surrounding the internal walls 55, may be substantially solid or may be porous.

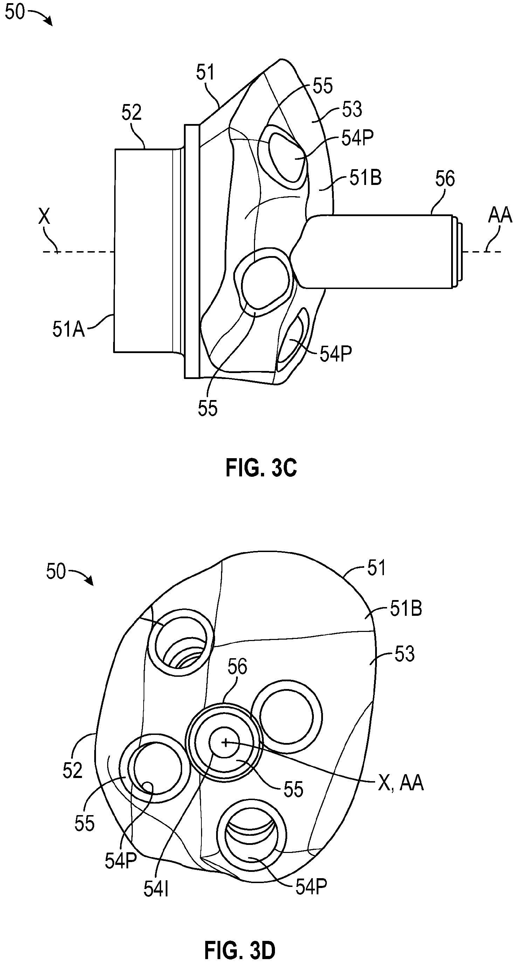

The implant 50 may include one or more fixation members 56. Each fixation member 56 may be dimensioned to secure the implant 50 to bone and/or other tissue at the surgical site. The fixation member 56 may be arranged to oppose shear forces experienced by the implant 50 subsequent to implantation. Various fixation members may be utilized, such as posts, pegs, keels, etc. In the implementation of Figures 3A and 3C-3E, the fixation member 56 may be an elongated post. The fixation member 56 may extend along a fixation (e.g., longitudinal) axis AA. One of the internal walls 55 may establish the fixation member 56 (see, e.g., Figures 3D-3E). The fixation member 56 may be dimensioned to extend from the rear face 51B of the implant 50. An aperture 54 may be dimensioned to extend through the fixation member 56, such as the intermediate aperture 541. The fixation axis AA may be collinear or otherwise parallel to the implant axis X. In the implementation of Figure 4, implant 150 may include a fixation member 156 dimensioned such that the fixation axis AA may be offset from, or transverse to, the implant axis X. In the implementation of Figure 5, implant 250 may include a plurality of fixation members 256. The fixation members 256 may be dimensioned such that each fixation axis AA may be offset from, or may be transverse to, the implant axis X. Each fixation member 56/156/256 may be dimensioned such that the fixation member 56/156/256 may be offset from a combined volume of the peripheral apertures 54P/154P/254P.

Referring to Figure 3E, an articulation component 57 may be configured to mate with, or otherwise be secured to, the implant 50 (shown in dashed lines). The articulation component 57 may be secured to the base plate 52 adjacent to the front face 51A of the implant model 32. Various techniques may be utilized to secure the articulation component 57 to the implant 50, such as a Morse taper connection and/or one or more fasteners. The articulation component 57 may include an articular surface 57A dimensioned to cooperate with an adjacent bone or implant. An articular surface 57A may be arranged to mate with an opposed articular surface SA (shown in dashed lines). The articular surface SA may be established by the adjacent bone or implant. In implementations, the adjacent bone may be a humerus that opposes a glenoid. The articular surface 57A may have various geometries including a substantially convex or concave geometry. The articulation component 57 may be a glenosphere having a substantially convex articular surface 57A. In other implementations, the articulation component 57 may be omitted, and the front face 51A of the implant 50 may establish the articular surface 57A. The articulation component 57 may be associated with a respective virtual implant model 32 in the form of a virtual articulation component.

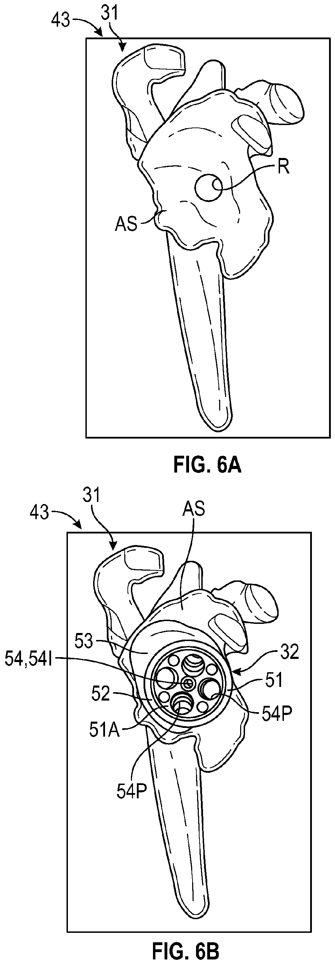

Referring to Figures 6A-6D, with continuing reference to Figures 2 and 3A-3F, the spatial module 39 may be configured to position the implant model 32 relative to the bone model 31. The bone model 31 may be associated with a scapula of a patient. In the implementation of Figure 6A, at least one recess R may be established along the articular surface AS and/or another surface of the bone model 31. The articular surface AS may be associated with a glenoid. The recess R may be dimensioned to at least partially receive a respective fixation member 56 for securing the implant to the respective bone (see, e.g., Figure 6D).

Referring to Figure 7, with continuing reference to Figure 2, the user interface 43 may include a first display window 44-1. Although one display window 44 is shown, it should be understood that more than one display window 44 may be utilized in accordance with the teachings disclosed herein. Geometric objects, including selected virtual bone model(s) 31, implant model(s) 32, transfer model(s) 48 and/or other information relating to a surgical plan 33 may be displayed in one or more of the display windows 44. The user interface 43 may include one or more objects 46, which may be linked to or otherwise associated with the display window(s) 44. The objects 46 may include graphics such as menus, tabs, lists, entry fields and buttons accessible by user interaction, such as tabs 46T, buttons 46B, drop-down lists 46L, menus 46M, directional indicators 46D, sliders 46S, translational button 46TR, rotation button 46R and/or tilt button 46TI. The objects 46 may include a pointer 46P associated with a mouse or other input device. In implementations, one or more entries may be specified in respective entry fields, including any parameters associated with the lists 46L. A surgeon or clinical user may interact with the user interface 43 to retrieve, view, edit, store, etc., various aspects of a surgical plan 33, such as the selected bone model(s) 31, implant model(s) 32 and/or transfer model(s) 48. The surgeon or clinical user may interact with the objects 46, display window 44-1 and/or another portion of the user interface 43 to select one or more implant models 32. Various parameters may be utilized to select the implant model 32, such as surgical procedure, anatomy, type, size, etc. The surgeon or clinical user may interact with the objects 46, directly with the display window(s) 44 (e.g., with the pointer 46P) and/or another portion of the user interface 43 to adjust or otherwise define the position of the implant model 332.

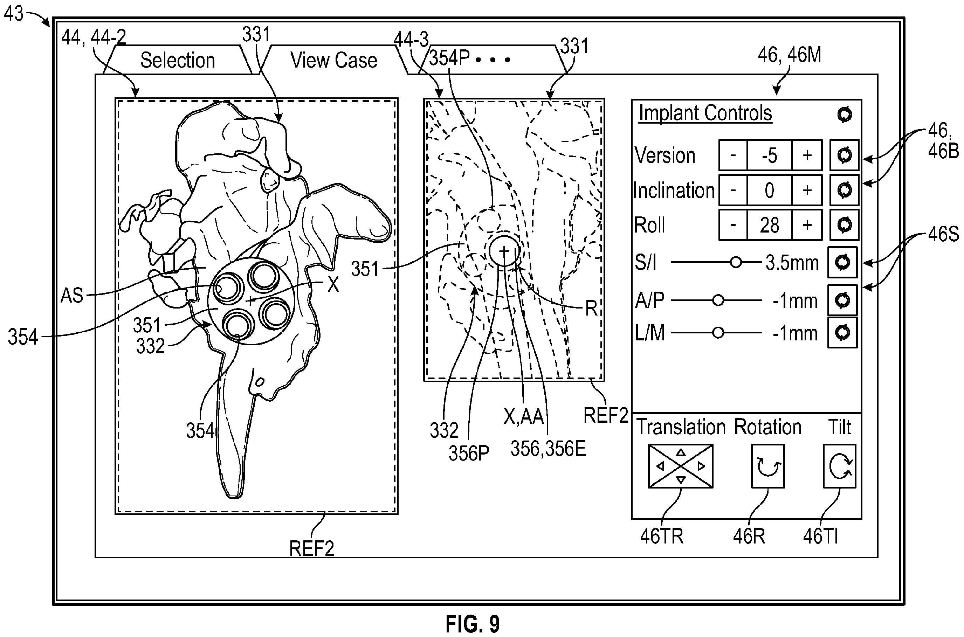

In the implementation of Figure 9, the planning environment 27 may be configured to define a position of a virtual implant model 332 relative to a virtual bone model 331. The planning environment 27 may be configured to cause the implant model 332 to be displayed in the graphical user interface 43 relative to the bone model 331. The spatial module 39 may be configured to set a position of a main body 351 of the implant model 332 relative to the bone model 331.

Still referring to Figures 2 and 7, the planning environment 27 may be configured to define one or more aspects of the implant model 32, including a geometry and/or position of the implant model 32 relative to the bone model 31. The planning environment 27 may be configured to incorporate one or more fixation members 56 into the implant model 32. The planning environment 27 may be configured to define a geometry of each fixation member 56, including defining a size and/or shape of the fixation member 56. The teachings disclosed herein may be utilized to establish fixation members for treating any of the bones and joints disclosed herein, including the glenoid of a shoulder joint. The disclosed techniques may be utilized to establish fixation members for implants associated with other bones and joints, such an implant associated with a tibia plateau or hip, a stem of an implant insertable in a femur or humerus, etc.

The planning environment 27 may be configured to define a position of the fixation member 56 relative to the main body 51 of the implant model 32. In implementations, the planning environment 27 may be configured to define the position of the fixation member 56 relative to the main body 51 in response to moving the fixation member 56 along a first reference plane REF1, which may be transverse to the implant axis X. The first reference plane REF1 may intersect the peripheral apertures 54P of the implant model 32. In implementations, the first reference plane REF1 may extend along the front (e.g., lateral) face 51A of the implant model 32. The planning environment 27 may be configured to establish a default (e.g., starting) position of the fixation axis AA of the fixation member 56 to be along implant axis X (see, e.g., Figure 7). The fixation axis AA of the fixation member 56 may be substantially perpendicular to the first reference plane REF1. In other implementations, the fixation axis AA may be transvers to the first reference plane REF1.

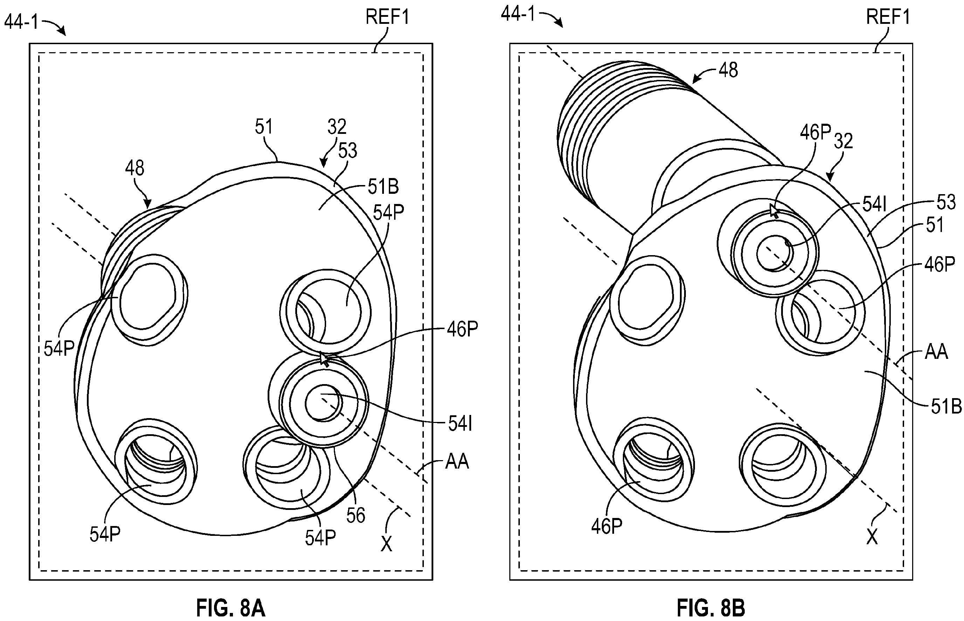

The fixation member 56 may be moveable along the reference plane REF1 such that a volume of the fixation member 56 may be spaced apart from a combined volume of the peripheral aperture(s) 46P of the implant model 32 (see, e.g., Figures 8A-8B). The spatial module 39 may be configured to move the fixation member(s) 56 relative to the reference plane REF1 in response to user interaction with the graphical user interface 43. The surgeon or clinical user may interact with the objects 46, directly with the display window(s) 44 (e.g., with the pointer 46P) and/or another portion of the user interface 43 to adjust or otherwise define the (e.g., default) position of each fixation member 56. The spatial module 39 may be configured to adjust or otherwise define the trajectory of a guide element GE and/or transfer model 48 with the fixation axis AA of the associated fixation member 56 in response to moving the fixation member 56 relative to the reference plane REF1. A physical instance of the guide element GE may be utilized to position a transfer guide associated with the transfer model 48 and/or an implant associated with the implant model 32 according to a surgical plan 33.

The spatial module 39 may be configured to define a trajectory associated with each respective aperture 54. The trajectory may be established along an axis FA of the respective aperture 54. The trajectory may be associated with a respective fastener F insertable through the aperture 54 such that a volume of the fastener F may be offset from the volume of the fixation member(s) 56 (see, e.g., Figure 3E).

The planning environment 27 may be configured to generate a configuration 47 associated with a physical implant model representative of the virtual implant model 32 according to the defined geometry, including the fixation member(s) 56. The planning environment 27 may be configured to generate a surgical plan 33 associated with the defined position of the virtual implant model 32 and the defined trajectory of each associated fastener(s) F.

Referring to Figure 9, with continuing reference to Figure 2, the planning environment 27 may be configured to cause an implant model 332 to be displayed in one or more display windows 44 of the graphical user interface 43 relative to a bone model 331. The bone model 331 may be associated with any of the bones and joints disclosed herein, such as a scapula. The user interface 43 may include second and third display windows 44-2, 44-3. The display windows 44-2, 44-3 may be associated with one or more objects 46 of the user interface 43.

The planning environment 27 may be configured to define a position of the implant model 332 relative to the virtual bone model 331 utilizing any of the techniques disclosed herein. The virtual implant model 332 may incorporate any of the features disclosed herein. In implementations, the implant model 332 may include a rear face 351B having a patient-specific contour dimensioned to substantially follow a contour of an articular surface AS associated with the bone model 331 (see, e.g., Fig. 3E).

The implant model 332 may include at least one fixation member 356. The planning environment 27 may be configured to define a geometry, including one or more dimensions, of each fixation member 356 relative to an anatomy of the patient to improve fixation of the associated implant. The fixation member 356 may be dimensioned to extend outwardly from the rear face 351B of the implant model 332. The fixation member 356 may include any of the geometries disclosed herein, such as an elongated post. Each fixation member 356 may extend along a fixation axis AA. The fixation member 356 may be dimensioned such that the fixation axis AA may be collinear with or otherwise parallel to an implant axis X of the implant model 332. The fixation member 356 may be dimensioned such that the fixation axis AA may be offset from, or may be transverse to, the implant axis X utilizing any of the techniques disclosed herein.

The spatial module 39 may be configured to set a position of the main body 351 of the implant model 332 relative to the bone model 331 and/or a second reference plane REF2. The reference plane REF2 may be established relative to one or more directions and/or landmarks of the anatomy. In implementations, the reference plane REF2 may extend in a superior-inferior (S/I) direction and/or anterior-posterior (A/P) direction of the anatomy. The spatial module 39 may be configured to substantially align the first reference plane REF1 (Figure 7) and the second reference plane REF2 to each other. The spatial module 39 may be configured to adjust the position and/or orientation of the implant axis X and/or fixation axis AA in response to translating, rotating and/or tilting the implant model 332. The surgeon or clinical user may interact with the user interface 43 to adjust or otherwise define the roll, inclination and/or version of the implant model 332.

The spatial module 39 may be configured to adjust the position of the implant model 332 in one or more directions associated with the anatomy. The directions may include the superior-inferior (S/I) direction, anterior-posterior (A/P) direction and/or lateral-medial (L/M) direction of the anatomy. The spatial module 39 may be configured to adjust a position of each aperture 354 in response to rotating the implant model 332 about the respective implant axis X. The surgeon or clinical user may interact with the objects 46, directly with the display window(s) 44-2, 44-3 and/or another portion of the user interface 43 to adjust or otherwise define the position and/or orientation of the implant model 332 relative to the bone model 331 and/or second reference plane REF2. The surgeon or clinical user may interact with the user interface 43 to position the fixation member(s) 356 and/or apertures 354 for receiving respective fasteners to improve fixation based on a geometry and/or quality of the respective bone.

The planning environment 27 may be configured to adjust or otherwise define a position and/or orientation of the fixation member 356 such that a distal (e.g., free) end 356E of the fixation member 356 may perforate one or more (e.g., medial) surfaces of the bone model 331 when the implant model 332 is seated against another (e.g., articular or lateral) surface of the bone model 331. The perforated surface(s) may be associated with a cortical wall of the bone, which in implementations may be associated with a glenoid vault of the scapula.

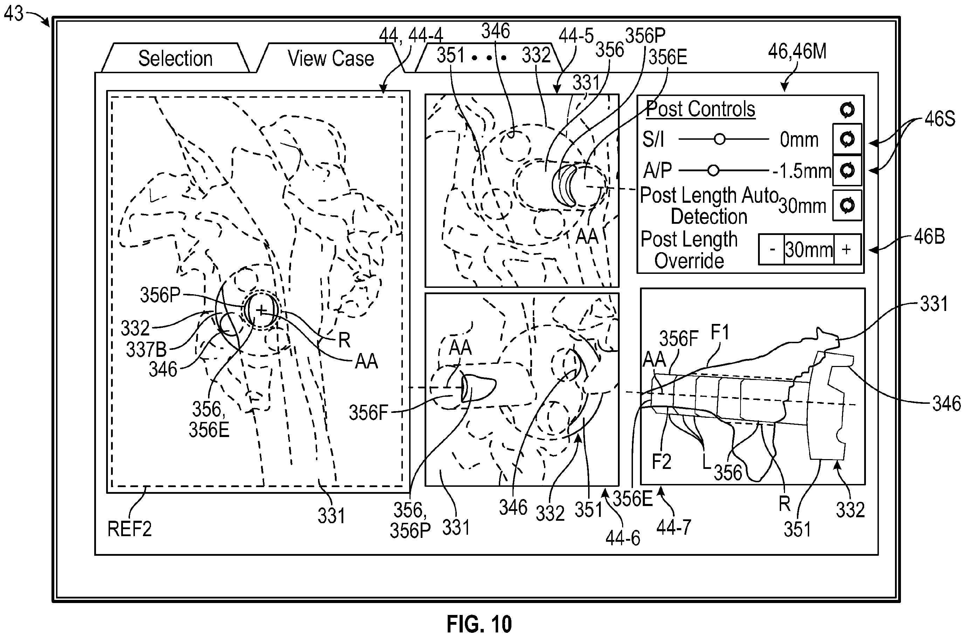

Referring to Figure 10, with continuing reference to Figures 2 and 9, the user interface 43 may include one or more display windows 44, such as fourth through seventh display windows 44-4 to 44-7. The display windows 44-4 to 44-7 may be associated with one or more objects 46 of the user interface 43. The planning environment 27 may be configured to define dimension(s) (e.g., length, width, etc.) of the fixation member 356 such that the free end 356E may perforate an external surface of the bone model 331. The dimension(s) of each fixation member 356 may be defined automatically and/or in response to user interaction with the user interface 43.

The planning environment 27 may be configured to adjust or otherwise define a length L of each fixation member 356. The length L may be defined relative to the rear face 351B of the main body 351 of the implant model 332. The planning environment 27 may be configured to increase and/or decrease the length L automatically and/or in response to user interaction. In implementations, planning environment 27 may be configured to generate a set of selectable lengths L of the fixation member 356. The user interface 43 may be configured to display the set of selectable lengths L relative to the bone model 331. The surgeon or clinical user may interact with the user interface 43 to select or otherwise define the length L and/or associated depth of the fixation member 356 such that a distal end portion 356P of the fixation member 356 may perforate an external surface of the bone model 331 when the implant model 332 is seated against another (e.g., articular) surface of the bone model 331.

The comparison module 40 may be configured to define a length L of the fixation member 356 such that the distal end portion 356P of the fixation member 356 may perforate one or more (e.g., first and/or second) faces on opposite sides of a volume of the virtual bone model 331 when the virtual implant model 332 is situated in a defined position relative to the virtual bone model 331. The surgeon or clinical user may interact with the user interface 43 to define the position, orientation and/or geometry of the fixation member 356 such that a first portion of the free end 356E may perforate a first face F1 and/or a second portion of the free end 356E may perforate a second face F2 of the bone model 331. The first and second faces F1, F2 may be anterior and posterior faces that may cooperate to bound a volume associated with a glenoid vault of the scapula.

The comparison module 40 may be configured to generate a suggested (e.g., detected or default) length L of the fixation member 356 that may be sufficient for the end portion 356P of the fixation member 356 to perforate the external surface of the bone model 331. The surgeon or clinical user may interact with the user interface 43 to adjust (e.g., override) the suggested length L. The planning environment 27 may be configured generate a configuration 47 associated with a physical implant model that may be representative of the virtual implant model 332 according to the defined length.

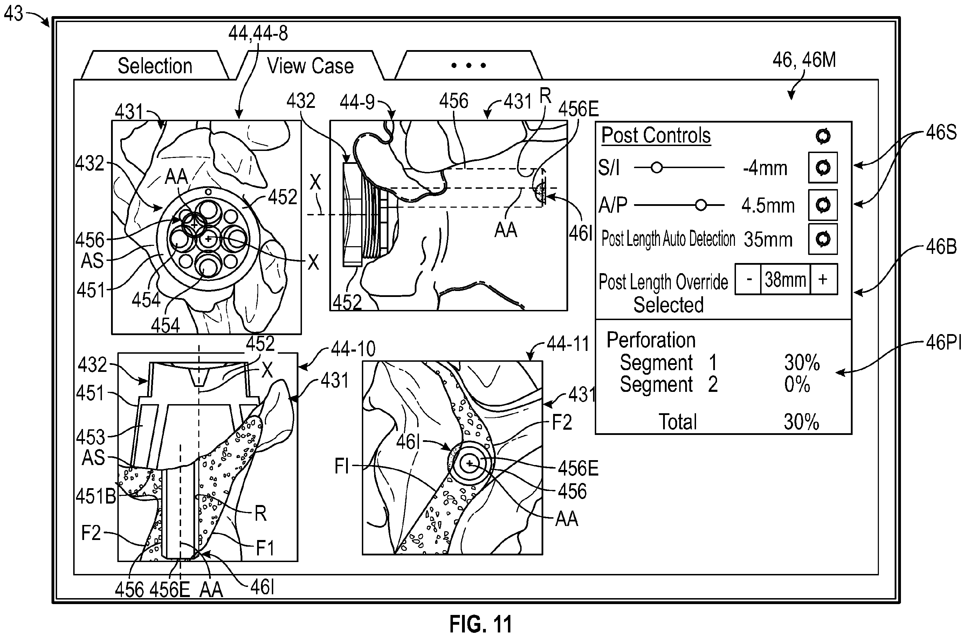

Referring to Figure 11, with continuing reference to Figures 2 and 9-10, the comparison module 40 may be configured to generate one or more indicators associated with fixation member(s) 456. The indicators may be utilized to communicate clinically useful information to the surgeon or clinical user. Various techniques may be utilized to establish the indicators. In the implementation of Figure 11, the user interface 43 may include one or more display windows 44, such as eighth through eleventh display windows 44-8 to 44-11, which may be associated with one or more objects 46 of the user interface 43. The indicators may be generated automatically and/or in response to user interaction with the user interface 43 and/or another portion of the planning system 20. The comparison module 40 may be configured to generate one or more indicators in response to one or more predetermined conditions being met, such as perforation of the respective fixation member 456 relative to an external surface of a bone model 431. The comparison module 40 may be configured to cause the display module 37 to display the indicator(s) in one or more of the display windows 44 based on a position of the respective fixation member 456 relative to the external surface of the bone model 431.

Various indicators may be utilized, including textual indicators and/or geometrical indicators that may establish a visual contrast with adjacent portions of the fixation member 456 and/or bone model 431. The indicators may include one or more characteristics that may be distinct from the bone model 431 and/or implant model 432, including different visual characteristics (e.g., shapes, patterns, colors, shades, etc.). The indicators may be established according to one or more visual or color schemes. The visual contrast may assist the surgeon or clinical user in identifying a condition associated with the indicator(s). Each indicator may be associated with an object 46 of the user interface 43. In implementations, the user interface 43 may include a display field 46PI that may present values and/or other information associated with perforation of one or more segments that establish a periphery of the fixation member 456.

The user interface 43 may include graphical indicator(s) 461 that may establish a visual contrast utilizing different colors, shades, hatching, textures, etc. The comparison module 40 may be configured to generate one or more indicators 46I associated with a first portion of a distal (e.g., free) end 456E that may perforate a first (e.g., anterior) face F1 and/or a second portion of the free end 456E that may perforate a second (e.g., posterior) face F2 of the bone model 431. The display field 46PI may include a set of indicators, such as a first indicator associated with a first percentage of the periphery of the fixation member 456 that may perforate the first (e.g., anterior) face F1 (e.g., segment 1 of the display field 46PI) and/or a second indicator associated with a second percentage of the periphery that may perforate the second (e.g., posterior) face F2 (e.g., segment 2 of the display field 46PI). The display field 46PI may include a total of the percentages of the periphery of the fixation member 456 that may perforate the external surface of the bone model 431. The planning environment 27 may be configured to define the length L and/or width of the fixation member 456 such that the percentage of the periphery of the fixation member 456 that perforates the cortex of the associated bone exceeds a predetermined threshold (e.g., 25 percent).

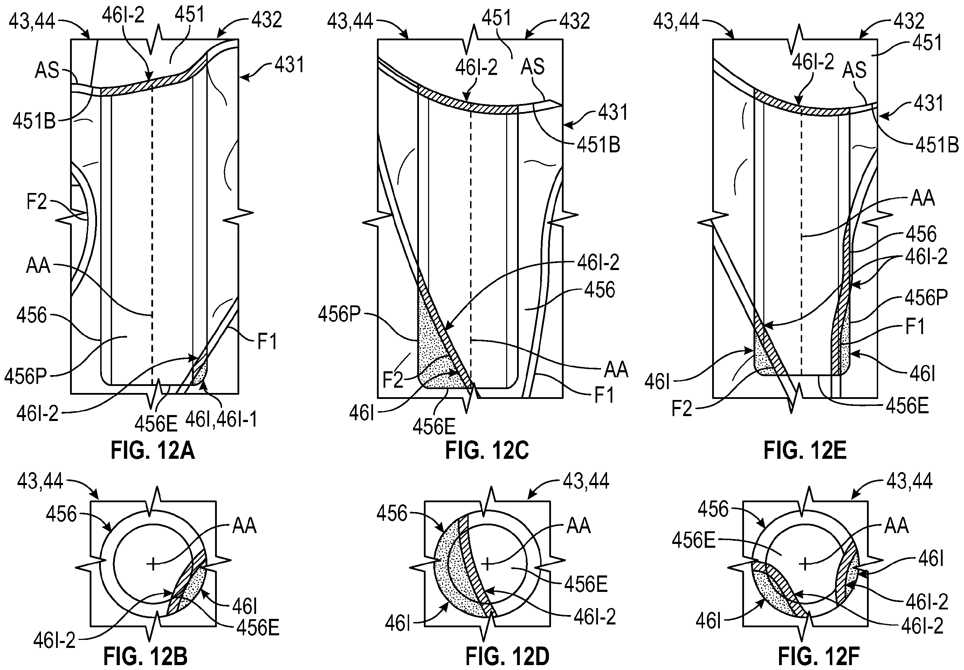

In the implementation of Figures 12A-12B, the display module 38 may be configured to display an indicator 46I associated with the fixation member 456 perforating the first (e.g., anterior) face F1 of the bone model 431. In the implementation of Figures 12C-12D, the display module 38 may be configured to display an indicator 46I associated with the fixation member 456 perforating the second (e.g., posterior) face F2 of the bone model 431. In the implementation of Figures 12E-12F, the display module 38 may be configured to display a set of indicators 46I associated with the fixation member 456 perforating the first and second faces F1, F2 of the bone model 431 along the periphery of the end portion 456P of the fixation member 456. Perforating the bone on opposite sides of the periphery of the fixation member 456 may achieve a clamping action for improving fixation. A portion of the distal end 456E of the fixation member 456 between the faces F1, F2 may be situated in a volume of the bone model 431. In the implementation of Figures 12A-12F, the planning display module 38 may be configured to generate indicator(s) associated with perforation of the fixation member 456 (e.g., indicators 461-1) and may be configured to generate other indicators(s) associated with the fixation member 456 situated in a portion of the bone model 431 associated with cortical bone (e.g., indicators 461-2). Utilizing the techniques disclosed herein, a physical implant associated with the virtual implant model 332 may achieve bi-cortical fixation, which may improve a stability of the implant and reduce a likelihood of loosening of the implant over time.

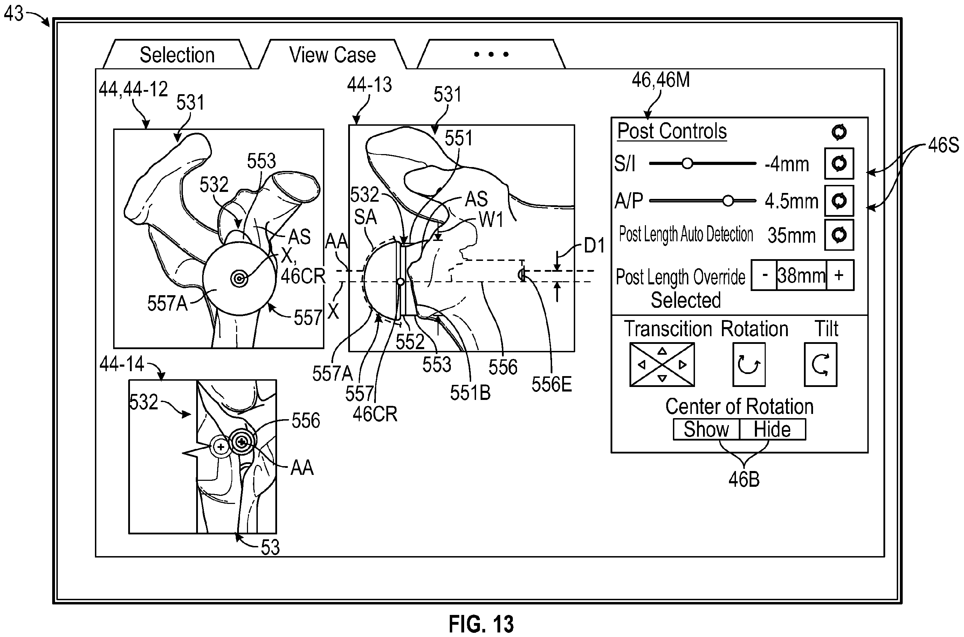

Referring to Figure 13, with continuing reference to Figure 2, the user interface 43 may define the position and/or geometry of an implant model 532 based on a center of rotation 46CR associated with a joint. The center of rotation 46CR may be associated with an adjacent bone or implant (e.g., humerus or humeral implant). The user interface 43 may include one or more display windows 44, such as twelfth through fourteen display windows 44-12 to 44-14, which may be associated with one or more objects 46 of the user interface 43. The comparison module 40 may be configured to cause the display module 37 to display the center of rotation 46CR.

An articular surface 557A associated with the implant model 532 may establish the center of rotation 46CR. The adjacent bone or implant may articulate relative to the center of rotation 46CR. The adjacent bone or implant may include an articular surface SA (shown in dashed lines in window 44-13), which may mate or otherwise cooperate with the articular surface 557A. The articular surface 557A may be established by an articulation component 557 coupled or otherwise positioned relative to the implant model 532. The articulation component 557 may be associated with a respective virtual implant model 32 in the form of a virtual articulation component. The implant model 532 and/or articulation component 557 may be positioned relative to a bone model 531 utilizing any of the techniques disclosed herein. The center of rotation CR may be substantially aligned with an implant axis X of the implant model 532. In other implementations, the center of rotation 46CR may be offset from the implant axis X.

The planning environment 27 may be configured to adjust or otherwise define a position and/or orientation of each fixation member 556 relative to the center of rotation 46CR. Each fixation member 556 may extend along a respective fixation axis AA. The spatial module 39 may be configured to position the virtual articulation component 557 relative to the virtual implant model 332 to establish the center of rotation 46CR associated with an adjacent implant or bone, which may mate with the articular surface 557A of the virtual articulation component 557.

The planning environment 27 may be configured to define the position of the fixation member 556 such that the fixation axis AA may be offset from the center of rotation 46CR (see, e.g., display window 44-13). A first distance D1 may be established between the fixation axis AA of the fixation member 556 and the center of rotation 46CR. A maximum width W1 may be established across a periphery of the main body 551 of the implant model 532, such as a periphery of the baseplate 552 or the augment 553. An offset ratio D1:W1 may be defined as the first distance D1 divided by the maximum width W1. The fixation axis AA may be offset from the center of rotation 46CR such that the offset ratio D1:W1 may be greater than zero. In implementations, the main body 551 and the fixation member 556 may be dimensioned such that the offset ratio D1:W1 may be equal to or greater than 0.10, or more narrowly equal to or greater than 0.25. The fixation member 556 may be spaced apart from a periphery of the main body 551 of the implant model 532 such that the offset ratio D1:W1 may be less than 1.0, or more narrowly equal to or less than 0.90. In implementations, the offset ratio D1:W1 may be equal to or less than 0.75. The offset ratios D1:W1 disclosed herein may improve fixation of the implant and range of motion associated with a center of rotation of the joint. In other implementations, the fixation axis AA may be substantially aligned with the center of rotation 46CR. The surgeon or clinical user may interact with the objects 46, directly with the display window(s) 44-12 to 44-14 and/or another portion of the user interface 43 to adjust or otherwise define the position and/or orientation of each fixation member 556 relative to the center of rotation 46CR, including based on various characteristics such as bone quality and geometry, which may improve fixation of the associated implant. The position, orientation and/or geometry of each fixation member 556 may be defined such that a free end 556E of the fixation member 556 may perforate one or more (e.g., medial) surfaces of the bone model 531 when the implant model 532 is seated against another (e.g., articular or lateral) surface of the bone model 531 utilizing any of the techniques disclosed herein.

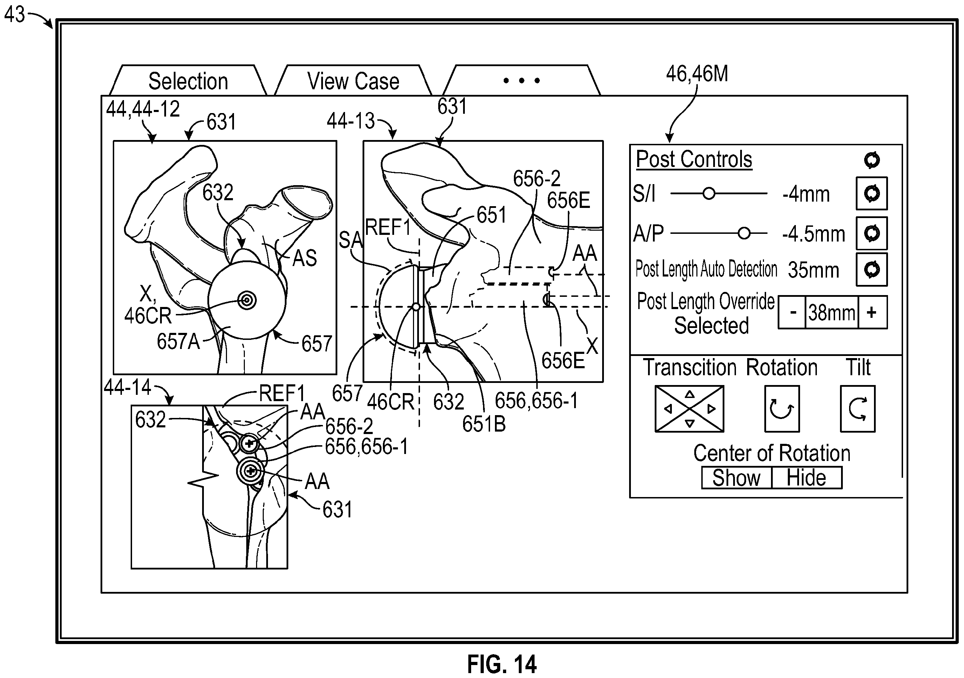

In the implementation of Figure 14, an implant model 632 may include two or more fixation members 656. The surgeon or clinical user may define the position, orientation and/or geometry of each fixation member 656 utilizing any of the techniques disclosed herein. The fixation members 656 may include a first fixation member 656-1 and a second fixation member 656-2, which may be defined relative to a first reference plane REF1. The spatial module 39 may be configured to move the first and second fixation members 656-1, 656-2 independently of each other and/or relative to the reference plane REF1 such that the fixation members 656-1, 656-2 may be spaced apart from each other (see, e.g., display windows 44-13, 44-14). In implementations, the fixation members 656-1, 656-2 may be offset in the superior/inferior direction (S/I), anterior/posterior (A/P) and/or medial/lateral (M/L) directions. At least one, more than one, or each of the fixation members 656 may be dimensioned to meet any the offset ratios D1:W1 disclosed herein. The offset ratios D1:W1 of the fixation members 656 may be the same or may differ from each other. Utilizing the teachings disclosed herein, the position and/or orientation of each fixation member 556/656 may be defined independently of the center of rotation 46CR, which may improve range of motion and fixation of the associated implant to bone or other tissue at the surgical site.

The planning environment 27 may be configured to adjust the length of the first fixation member 656-1 and the length of the second fixation member 656-2 independently of each other such that a free end 656E of the first fixation member 656-1 and/or a free end 656E of the second fixation member 656-2 may at least partially or completely perforate a (e.g., medial) surface the virtual bone model 631. The perforated surface may be established by a cortical wall of the bone model 631 spaced apart from the rear face 651B of the implant model 632. In implementations, the perforated surface may include an anterior and/or posterior face of the scapula, which may cooperate to bound a glenoid vault. A portion of each fixation member 656 may be dimensioned to extend through a region of cancellous bone associated with the bone model 631. Each fixation member 656 may be dimensioned to establish bi-cortical fixation when the implant model 632 is seated against articular surface AS of the bone model 531 by extending at least partially through a first cortical wall along the articular surface AS of the bone model 631 and a second cortical wall that may be established inwardly (e.g., medially) of the first cortical wall. The first and second cortical walls may be associated with cortical bone.

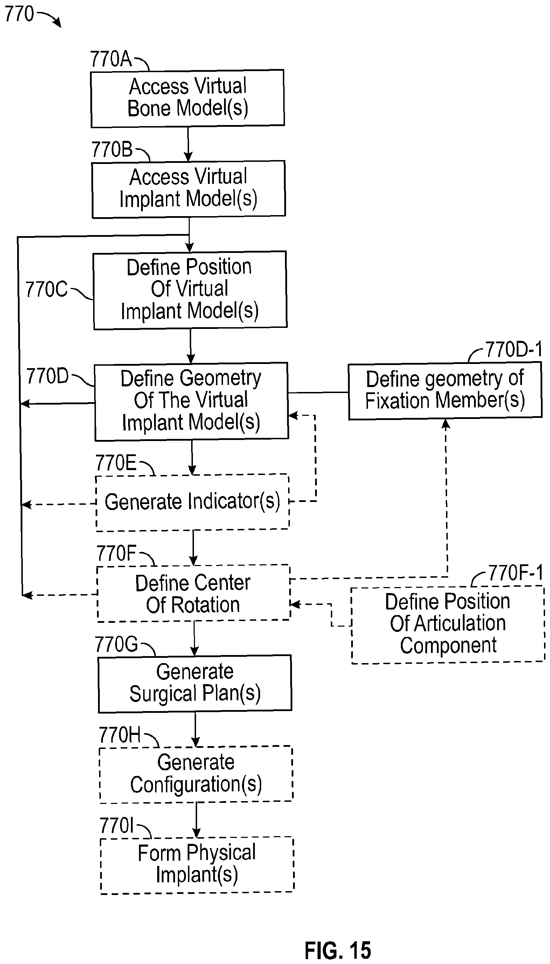

Figure 15 discloses a method of establishing an implant for a surgical procedure in a flowchart 770. The method may be utilized to perform an arthroplasty for restoring functionality to various bones and joints, including any of the bones and associated joints disclosed herein such as shoulder, ankle, hip, knee and elbow joints. In implementations, the method may be utilized to perform an arthroplasty for restoring functionality to shoulders having advanced cartilage disease, such as repairing bone defects along a glenoid. The method can be utilized with any of the implants and fixation members disclosed herein. Fewer or additional steps than are recited below could be performed within the scope of this disclosure, and the recited order of steps is not intended to limit this disclosure. It should be understood that the planning environment 27 and any of the associated modules may be configured to perform any of the features of the method 770.

Referring to Figure 2, with continuing reference to Figure 15, one or more virtual bone models 31 may be selected or otherwise accessed from memory at step 770A. The bone models 31 may include any of the bone models disclosed herein. Each bone model 31 may be associated with a bone of a respective patient or may be a bone model representative of a hypothetical case.

At step 770B, one or more virtual implant models 32 may be selected or otherwise accessed from memory. The implant models 32 may include any of the implant models disclosed herein. Each implant model 32 may have a patient-specific geometry associated with a respective patient or may have a non-patient specific geometry for treating two or more patients. In the implementation of Figures 3A-3E, the implant model 32 may include a main body 51 extending between a front face 51A and a rear face 51B. The implant model 32 may include one or more peripheral apertures 54P extending through the main body 51. Each of the peripheral apertures 54P may be dimensioned to receive a respective fastener F (Figure 3E). The implant model 32 may include at least one fixation member 56. Each fixation member 56 may extend from the rear face 51B of the implant model 32.

Referring to Figures 6A-6D, with continuing reference to Figures 2 and 15, a position of each implant model 32 may be defined relative to a respective bone model 31 at step 770C. Various techniques may be utilized to position the implant model 32, including any of the techniques disclosed herein. Step 770C may include translating, rotating and/or tilting the implant model 32 relative to the bone model 31 to establish a defined position and/or orientation of the implant model 32.

A geometry of each implant model 32 may be defined at step 770D. The geometry of the implant model 32 may be defined utilizing any of the techniques disclosed herein. Step 770D may occur prior to, concurrently with, and/or subsequent to defining a position and/or orientation of the implant model 32 at step 770C. Method 770 may include repeating one or more iterations of step 770C and/or step 770D prior to approval of a surgical plan 33 associated with the respective bone model(s) 31 and/or implant model(s) 32. Step 770D may include defining a quantity, position and/or orientation of aperture(s) 54 that may be dimensioned to extend at least partially or completely through the main body 51 of the implant model 32. Step 770D may include defining the geometry of the virtual implant model 32 such that the rear face 51B may be dimensioned to substantially follow a contour of an articular surface AS associated with the virtual bone model 31 (see, e.g., Figure 3E).

Step 770D may include defining a quantity and/or geometry of fixation member(s) 56 for each implant model 32 at step 770D-1. The quantity and/or geometry of the fixation member(s) 56 may be defined utilizing any of the techniques disclosed herein. Referring to Figures 7 and 8A-8B, step 770D-1 may include defining a position and/or orientation of the fixation member(s) 56 relative to the main body 51 of the implant model 32. The position and/or orientation of each fixation member 56 may be defined relative to the main body 51 in response to moving the respective fixation member 56 along the first reference plane REF1 such that a volume of the fixation member 56 may be spaced apart from a combined volume of the peripheral aperture(s) 54P of the implant model 32. Step 770D-1 may include permitting movement of the fixation member 56 outside of the combined volume of the peripheral apertures 54P, but may include blocking movement of the fixation member 56 within the combined volume of the peripheral apertures 54P such that an interference or overlap between the peripheral aperture(s) 54P and fixation member(s) 56 may be avoided. In implementations, the comparison module 40 may be configured to generate one or more warnings or indicators in response to interference between the fixation member 56 and peripheral apertures 54P, which may be displayed in the user interface 43.

Referring to Figures 9-10, with continuing reference to Figures 2 and 15, defining the position of the implant model 332 at step 770C may include defining a position and/or orientation of the fixation member(s) 356 relative to the bone model 331. Defining the geometry of the fixation member(s) 356 at step 770D-1 may include defining a length L of the respective fixation member 356 (see, e.g., Figure 10). The length L of each fixation member 356 may be defined utilizing any of the techniques disclosed herein. Step 770D-1 may include defining the L length of the fixation member 356 relative to the rear face 351B of the main body 351 of the implant model 332 such that a distal end portion 356P of the fixation member 356 may perforate an external surface of the bone model 331, which may be spaced apart from the main body 351 of the implant model 332 (see, e.g., Figure 10). The L length of the fixation member 356 may be defined such that the distal end portion 356P of the fixation member 356 may perforate the first face F1 and/or the second face F2 of the bone model 331 when the virtual implant model 332 may be situated in the defined position. The first and second faces F1, F2 may be opposed to each other. The first and second faces F1, F2 may cooperate to bound the volume of the bone model 331. In implementations, the bone may be a scapula. The first and second faces F1, F2 may be anterior and posterior faces associated with a glenoid vault of the scapula.

Referring to Figures 11 and 12A-12F, with continuing reference to Figures 2, 10 and 15, one or more indicators may be generated at step 770E. Each indicator may be generated utilizing any of the techniques disclosed herein. In implementations, each indicator may be associated with a respective object 461. Step 770E may include generating at least one indicator 46I associated with a first portion of the free end 456E that may perforate the first (e.g., anterior) face F1 (e.g., Figures 11 and 12A-12B) and/or a second portion of the free end 456E that may perforate the second (e.g., posterior) face F2 (e.g., Figures 12C-12D). In implementations, step 770E may include generating a set of indicators 46I associated with the first portion of the free end 456E that may perforate the first face F1 and/or the second portion of the free end 456E that may perforate the second face F2 when the implant model 432 is seated against the bone model 431 (e.g., Figures 12E-12F).

Referring to Figures 13-14, with continuing reference to Figures 2 and 15, a center of rotation associated with the implant model 532/632 may be defined at step 770F. The center of rotation may be associated with an adjacent implant or bone. Step 770F may include displaying the center of rotation 46CR relative to the implant model 532/632. Defining the geometry of each fixation member 556/656 at step 770D-1 may include defining the geometry of the respective fixation member 556/656 relative to the center of rotation 46CR. Method 770 may include repeating one or more iterations of step 770D-1 and/or step 770F prior to approval of a surgical plan 33 associated with the respective bone model(s) 531/631 and/or implant model(s) 532/632.

Step 770F may include defining a position of an articulation component 557/657 relative to the virtual implant model 532/632 to establish a center of rotation 46CR associated with an adjacent implant or bone. The adjacent implant or bone may include an articular surface SA that may mate with the articular surface 557A/657A of the articulation component 557/657. Defining the position of each fixation member 556/656 at step 770D-1 may occur such that an axis AA of the respective fixation member 556/656 may be offset from the center of rotation 46CR. The virtual implant model 632 may include two or more fixation members 656 each having an axis AA that may be offset from the center of rotation 46CR.

Referring to Figures 6D and 7, with continuing reference to Figures 2 and 15, one or more surgical plans 33 may be generated at step 770G. The surgical plan 33 may incorporate information associated with any of the parameters disclosed herein. The surgical plan 33 may include information specifying the position and/or orientation of the respective implant model(s) 32 relative to the bone model(s) 31 of a patient. Step 770G may include defining a trajectory associated with one or more fasteners F such that a volume of each fastener F may be offset from the volume of the fixation member(s). The trajectory may be associated with the axis FA of the fastener F (see, e.g., Figure 7). Step 770G may include generating the surgical plan 33 associated with the defined position of the virtual implant model 32 and the defined trajectory of the fastener(s) F. The trajectory of each fastener F may be defined according to the position and/or orientation of the implant model 32 and associated peripheral apertures 54P defined at step 770C (see, e.g., Figures 6B-6D, 7 and 8A-8B).

One or more configurations 47 may be generated at step 770H. Each configuration 47 may be associated with a physical implant representative of the implant model 32 according to the geometry of the implant model 32 defined at step 770D. In the implementation of Figure 3E, a rear face 51B of the implant 50 may be dimensioned to substantially follow a contour of an articular surface AS associated with the bone model 31. In implementations, a configuration 47 may be generated for a physical transfer guide representative of a transfer model 48 for positioning the physical implant.

The physical implant may be formed at step 770I. Step 770I may include forming the physical implant based on the configuration 47 generated at step 770H. The physical implant may be formed utilizing any of the techniques disclosed herein. Figures 3A-3E disclose an implementation of a physical implant 50.

The novel devices and methods of this disclosure provide versatility in dimensioning an orthopaedic implant. The implant may incorporate one or more fixation members for securing the implant to bone or other tissue. The surgeon or clinical user may select a position and/or dimensions(s) of the fixation member to achieve improved fixation based on a geometry of the bone, bone quality, bone density and other characteristics. Utilizing the techniques disclosed herein, the fixation member may achieve bi-cortical penetration, such as along an articular surface (e.g., glenoid face) of the bone and another surface of the bone spaced apart from the articular surface (e.g., medial cortex at the backside of the glenoid vault), which may improve fixation and reduce a likelihood of loosening of the implant. The fixation member may be dimensioned to perforate opposed faces along a medial portion of the bone, which may achieve a clamping action for improving fixation of the implant. A length of the fixation member may be increased, which may improve stability of the implant. The position of the fixation member and a center of rotation established by the implant may be defined independently, which may improve fixation of the implant and range of motion of a limb associated with implant.

Although the different non-limiting embodiments are illustrated as having specific components or steps, the embodiments of this disclosure are not limited to those particular combinations. It is possible to use some of the components or features from any of the non-limiting embodiments in combination with features or components from any of the other non-limiting embodiments.

It should be understood that like reference numerals identify corresponding or similar elements throughout the several drawings. It should further be understood that although a particular component arrangement is disclosed and illustrated in these exemplary embodiments, other arrangements could also benefit from the teachings of this disclosure.

The foregoing description shall be interpreted as illustrative and not in any limiting sense. A worker of ordinary skill in the art would understand that certain modifications could come within the scope of this disclosure. For these reasons, the following claims should be studied to determine the true scope and content of this disclosure.

## Claims

1. A system for planning a surgical procedure comprising:
a computing device including a processor coupled to memory, wherein the processor is configured to:
access a virtual bone model from the memory, the virtual bone model associated with a bone of a patient;
define a position of a virtual implant model relative to the virtual bone model, wherein the implant model includes a main body extending along an implant axis between a front face and a rear face, one or more peripheral apertures extending through the main body and dimensioned to receive a respective fastener, and at least one fixation member extending from the rear face;
define a geometry of the at least one fixation member, including defining a position of the at least one fixation member relative to the main body in response to moving the at least one fixation member along a reference plane transverse to the implant axis such that a volume of the at least one fixation member is spaced apart from a combined volume of the one or more peripheral apertures; and
generate a configuration file configured to be used by a manufacturing machine, the configuration file being associated with a physical implant model representative of the virtual implant model according to the defined geometry.

2. The system as recited in claim 1, wherein the processor is configured to:
define a geometry of the virtual implant model such that the rear face is dimensioned to substantially follow a contour of an articular surface associated with the virtual bone model
and/or
wherein the processor is configured to:
define a trajectory associated with the fastener such that a volume of the fastener is offset from the volume of the at least one fixation member; and
generate a surgical plan associated with the defined position of the virtual implant model and the defined trajectory.

3. The system as recited in any of the previous claims, wherein the processor is configured to:
cause the virtual implant model to be displayed in a graphical user interface relative to the virtual bone model;
set a position of the main body relative to the virtual bone model; and
move the at least one fixation member relative to the reference plane in response to user interaction with the graphical user interface.

4. The system as recited in any of the previous claims, wherein the processor is configured to:
adjust a length of the at least one fixation member relative to the rear face of the main body.

5. The system as recited in the previous claim, wherein the bone is a scapula, and the processor is configured to:
adjust the length such that a free end of the at least one fixation member perforates an anterior face and a posterior face that cooperate to bound a volume associated with a glenoid vault of the scapula.

6. The system as recited in the previous claim, wherein the processor is configured to:
generate at least one indicator associated with a first portion of the free end that perforates the anterior face and/or a second portion of the free end that perforates the posterior face, and
wherein the at least one indicator may include a first indicator associated with a first percentage of a periphery of the at least one fixation member that perforates the anterior face and/or a second indicator associated with a second percentage of the periphery that perforates the posterior face.

7. The system as recited in any of the previous claims, wherein the at least one fixation member extends along a fixation axis, and the processor is configured to:
position a virtual articulation component relative to the virtual implant model to establish a center of rotation associated with an adjacent implant or bone that mates with an articular surface of the virtual articulation component; and
define the position of the at least one fixation member such that the fixation axis is offset from the center of rotation, and
wherein:
the articular surface may have a substantially convex geometry; and/or
may have a perimeter of the main body has a substantially circular geometry.

8. The system as recited in the previous claim, wherein:
the at least one fixation member includes a first fixation member and a second fixation member; and
the processor is configured to move the first and second fixation members independently of each other relative to the reference plane such that the first and second fixation members are spaced apart, and
wherein the processor may be configured to:
adjust a length of the first fixation member and a length of the second fixation member independently of each other such that a free end of the first fixation member and/or a free end of the second fixation member perforates a cortical wall associated with the virtual bone model.

9. A system for planning a surgical procedure comprising:
a computing device including a processor coupled to memory, wherein the processor is configured to:
access a virtual bone model from the memory, the virtual bone model associated with a bone of a patient;
define a position of a virtual implant model relative to the virtual bone model, wherein the virtual implant model includes one or more peripheral apertures extending through a main body and a fixation member extending from a rear face of the main body;
define a length of the fixation member such that a distal end portion of the fixation member perforates first and second faces on opposite sides of a volume of the virtual bone model when the virtual implant model is situated in the defined position; and
generate a configuration file configured to be used by a manufacturing machine, the configuration file being associated with a physical implant model representative of the virtual implant model according to the defined length.

10. The system as recited in the previous claim, wherein the processor is configured to:
define a geometry of the virtual implant model such that the rear face is dimensioned to substantially follow a contour of an articular surface associated with the virtual bone model and/or
wherein:
the bone is a scapula; and
the first and second faces are anterior and posterior faces associated with a glenoid vault of the scapula.

11. A method, executed on a computing device, of establishing an implant for a surgical procedure comprising:
accessing a virtual bone model from memory, the virtual bone model associated with a bone of a patient;
defining a position of a virtual implant model relative to the virtual bone model, wherein the virtual implant model includes a main body extending between a front face and a rear face, one or more peripheral apertures extending through the main body and dimensioned to receive a respective fastener, and at least one fixation member extending from the rear face;
defining a geometry of the at least one fixation member, including defining a position of the at least one fixation member relative to the main body in response to moving the at least one fixation member along a reference plane such that a volume of the at least one fixation member is spaced apart from a combined volume of the one or more peripheral apertures; and
generating a configuration file configured to be used by a manufacturing machine, the configuration file being associated with a physical implant representative of the virtual implant model according to the defined geometry.

12. The method as recited in the previous claim, further comprising:
forming the physical implant based on the generated configuration file,
and/or
further comprising:
blocking movement of the at least one fixation member within the combined volume of the one or more peripheral apertures.

13. The method as recited in claim 11, further comprising:
defining a position of an articulation component relative to the virtual implant model to establish a center of rotation associated with an adjacent implant or bone that mates with an articular surface of the articulation component; and
the step of defining the position of the at least one fixation member occurs such that an axis of the at least one fixation member is offset from the center of rotation.

14. The method as recited in claim 11, wherein:
the step of defining the geometry of the at least one fixation member includes defining a length of the at least one fixation member relative to the rear face of the main body such that a distal end portion of the at least one fixation member perforates opposed first and second faces that cooperate to bound the volume of the virtual bone model when the virtual implant model is situated in the defined position.

15. The method as recited in the previous claim, wherein:
the bone is a scapula; and
the first and second faces are anterior and posterior faces associated with a glenoid vault of the scapula.
